# EUROPEAN PATENT SPECIFICATION

(11) **EP 4 329 693 B1**
(45) Date of publication and mention of the grant of the patent: **08.10.2025**
(21) Application number: 22722613.1
(22) Date of filing: 20.04.2022
(51) Int. Cl.: A61F 13/84, A61F 13/551

(54) **PACKAGED ABSORBENT ARTICLES**
VERPACKTE SAUGFÄHIGE ARTIKEL
ARTICLES ABSORBANTS EMBALLÉS

(30) Priority: 30.04.2021 US 202163181986 P
(43) Date of publication of application: 06.03.2024
(73) Proprietor: The Procter & Gamble Company, Cincinnati, OH 45202 (US)
(72) Inventor: RIEDEMAN, James Steven, Cincinnati, Ohio 45202 (US); BETANCOURT, Jose Enrique, Cincinnati, Ohio 45202 (US)
(74) Representative: P&G Patent Germany
(86) International application number: PCT/US2022/025446
(87) International publication number: WO 2022/231892

(56) References cited:
- WO-A1-2015/146891
- US-A1- 2018 289 564
- US-B2- 8 034 740

## Description

### FIELD

The present disclosure relates to one or more packaged absorbent articles, wherein the absorbent articles include a perfume composition.

### BACKGROUND

Non-fragile, compressible consumer products such as disposable absorbent articles (*e.g*., diapers and training pants, disposable adult incontinence pants and feminine hygiene pads) are often packaged and sold at retail (*i.e.,* placed on display and for sale in a retail store) in soft packages formed from flexible packaging material. At least a portion of the packaging material is typically printed with one or more graphics - such as brand identifiers, aesthetically pleasing designs, depictions of the enclosed absorbent article, directions for use of the absorbent article, and/or other consumer information. The graphics are typically formed from the deposition of ink onto the packaging material.

US2018289564A1 relates to a package of absorbent articles formed made of flexible polymeric film. The package has a line of weakness to open the package and to demarcate a package base and a package hood. The package hood may serve as a package reclosure device.

WO2015146891A1 discusses absorbent articles individually wrapped in a wrapping sheet and packages containing multiple of the wrapped articles. A fragrance is applied to each absorbent article. At least one side surface of the bag material is provided with holes through which the fragrance can pass.

US8034740B2 discloses a moisture- or protein-adsorbability imparting agent. The agent comprises a porous silica having a hexagonal pore structure, an average pore size of from 0.8 to 20 nm, an average particle size of 50 nm to 100 µm, a specific surface area of from 400 to 2000 m2/g, and a pore volume of from 0.1 to 3.0 cm3/g.

Absorbent articles are commonly used to absorb and retain bodily fluids and other exudates excreted by the human body. Such exudates may be, or may be perceived as, malodourous. Therefore, methods and materials for controlling and reducing malodors in absorbent articles have been developed. Fragrance materials have been used for this purpose in absorbent articles. Fragrance materials often include volatile components that evaporate into the environment surrounding the absorbent article and cover or block the perception of malodors. Certain volatile components may interact with packaging materials, inks, and/or components of inks, causing inks to soften and graphics to scuff, smear, or become unattractive and/or illegible. Therefore, packaged absorbent articles including perfume compositions should be improved.

### SUMMARY

Aspects of the present disclosure solve some or all or the problems discussed above by providing, in part, a package of one or more absorbent articles according to the claims.

### BRIEF DESCRIPTION OF THE DRAWINGS

The above-mentioned and other features and advantages of the present disclosure, and the manner of attaining them, will become more apparent and the disclosure itself will be better understood by reference to the following description of example forms of the disclosure taken in conjunction with the accompanying drawings, wherein:
Fig. 1 is a plan view of an example absorbent article in the form of a taped diaper, garment-facing surface facing the viewer, in a flat laid-out state;
Fig. 2 is a plan view of the example absorbent article of Fig. 1, wearer-facing surface facing the viewer, in a flat laid-out state;
Fig. 3 is a front perspective view of the absorbent article of Figs. 1 and 2 in a fastened position;
Fig. 4 is a front perspective view of an absorbent article in the form of a pant;
Fig. 5 is a rear perspective view of the absorbent article of Fig. 4;
Fig. 6 is a plan view of the absorbent article of Fig. 4, laid flat, with a garment-facing surface facing the viewer;
Fig. 7 is a cross-sectional view of the absorbent article taken about line 7-7 of Fig. 6;
Fig. 8 is a cross-sectional view of the absorbent article taken about line 8-8 of Fig. 6;
Fig. 9 is a plan view of an example absorbent core or an absorbent article;
Fig. 10 is a cross-sectional view, taken about line 10-10, of the absorbent core of Fig. 9;
Fig. 11 is a cross-sectional view, taken about line 11-11, of the absorbent core of Fig. 10;
Fig. 12 is a plan view of an example absorbent article of the present disclosure that is a sanitary napkin;
Fig. 13 is a side view of a stack of a plurality of absorbent articles disposed within a package of the present disclosure;
Fig. 14 is a cross-sectional view of a multi-layer film;
Fig. 15 is a cross-sectional view of a multi-layer film comprising tie layers;
Fig. 16 is a perspective view of a package of the present disclosure;
Fig. 17 is a schematic plan view of a package of the present disclosure constructed by a flow wrap process;
Fig. 18 is a schematic illustration of the Diffusion Rate Test Method disclosed herein; and
Fig. 19 is a graph of the diffusion rate of the perfume composition of Comparative Example 1 through various packaging materials.

### DETAILED DESCRIPTION

Various non-limiting forms of the present disclosure will now be described to provide an overall understanding of the principles of the structure, function, manufacture, and use of the packaged absorbent articles disclosed herein. One or more examples of these non-limiting forms are illustrated in the accompanying drawings. Those of ordinary skill in the art will understand that the packaged absorbent articles described herein and illustrated in the accompanying drawings are non-limiting example forms. The features illustrated or described in connection with one non-limiting form may be combined with the features of other non-limiting forms. Such modifications and variations are intended to be included within the scope of the present disclosure.

### General Description of an Absorbent Article

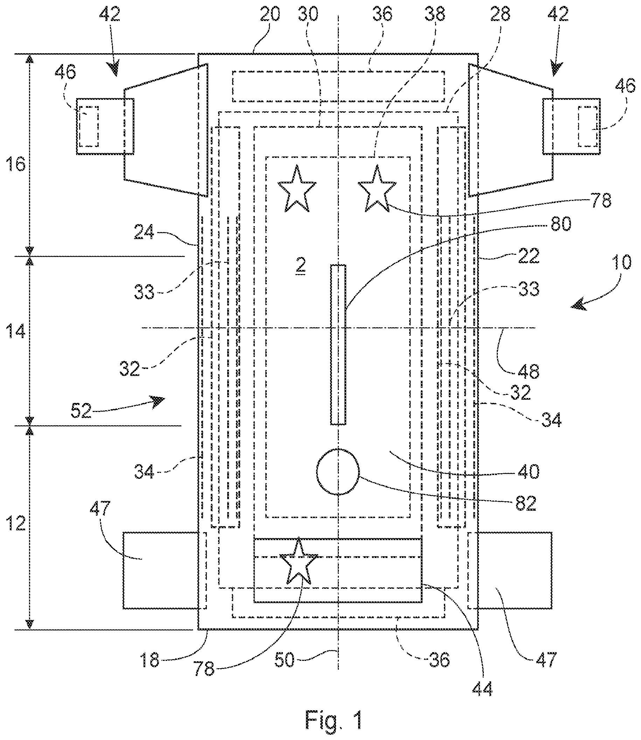

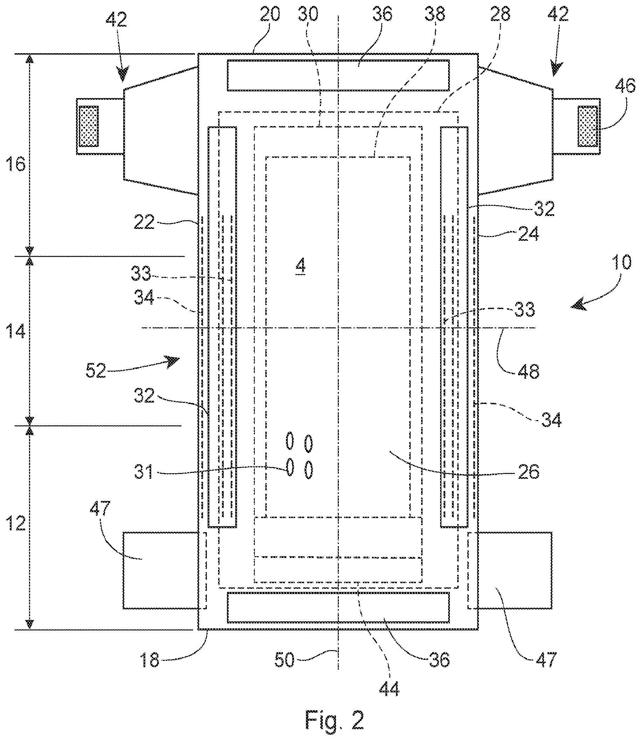

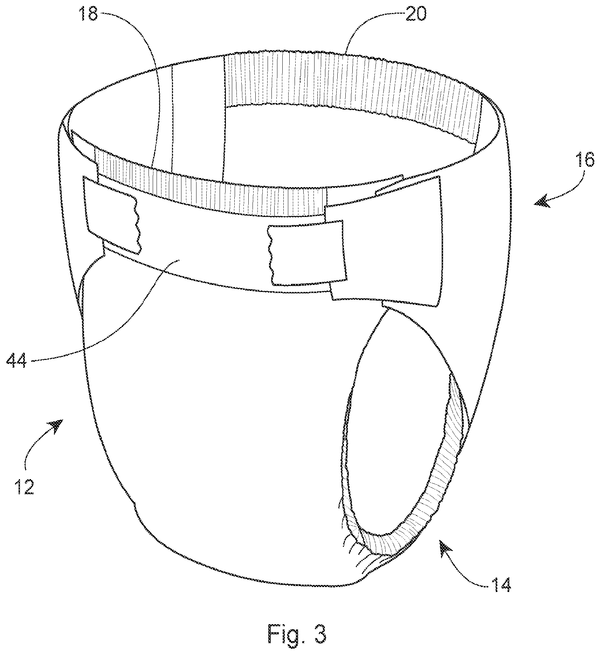

An example absorbent article 10 according to the present disclosure, shown in the form of a taped diaper, is represented in Figs. 1-3. Fig. 1 is a plan view of the example absorbent article 10, garment-facing surface 2 facing the viewer in a flat, laid-out state (i.e., no elastic contraction). Fig. 2 is a plan view of the example absorbent article 10 of Fig. 1, wearer-facing surface 4 facing the viewer in a flat, laid-out state. Fig. 3 is a front perspective view of the absorbent article 10 of Figs. 1 and 2 in a fastened configuration. The absorbent article 10 of Figs. 1-3 is shown for illustration purposes only as the present disclosure may be used for making a wide variety of diapers, including adult incontinence products, pants, or other absorbent articles, such as sanitary napkins and absorbent pads, for example.

The absorbent article 10 may comprise a front waist region 12, a crotch region 14, and a back waist region 16. The crotch region 14 may extend intermediate the front waist region 12 and the back waist region 16. The front wait region 12, the crotch region 14, and the back waist region 16 may each be 1/3 of the length of the absorbent article 10. The absorbent article 10 may comprise a front end edge 18, a back end edge 20 opposite to the front end edge 18, and longitudinally extending, transversely opposed side edges 22 and 24 defined by the chassis 52.

The absorbent article 10 may comprise a liquid permeable topsheet 26, a liquid impermeable backsheet 28, and an absorbent core 30 positioned at least partially intermediate the topsheet 26 and the backsheet 28. The absorbent article 10 may also comprise one or more pairs of barrier leg cuffs 32 with or without elastics 33, one or more pairs of leg elastics 34, one or more elastic waistbands 36, and/or one or more acquisition materials 38. The acquisition material or materials 38 may be positioned intermediate the topsheet 26 and the absorbent core 30. An outer cover material 40, such as a nonwoven material, may cover a garment-facing side of the backsheet 28. The absorbent article 10 may comprise back ears 42 in the back waist region 16. The back ears 42 may comprise fasteners 46 and may extend from the back waist region 16 of the absorbent article 10 and attach (using the fasteners 46) to the landing zone area or landing zone material 44 on a garment-facing portion of the front waist region 12 of the absorbent article 10. The absorbent article 10 may also have front ears 47 in the front waist region 12. The absorbent article 10 may have a central lateral (or transverse) axis 48 and a central longitudinal axis 50. The central lateral axis 48 extends perpendicular to the central longitudinal axis 50.

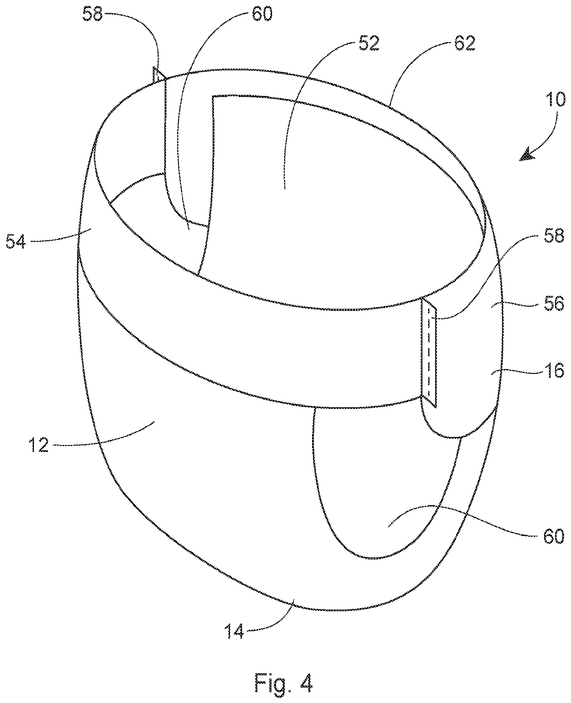

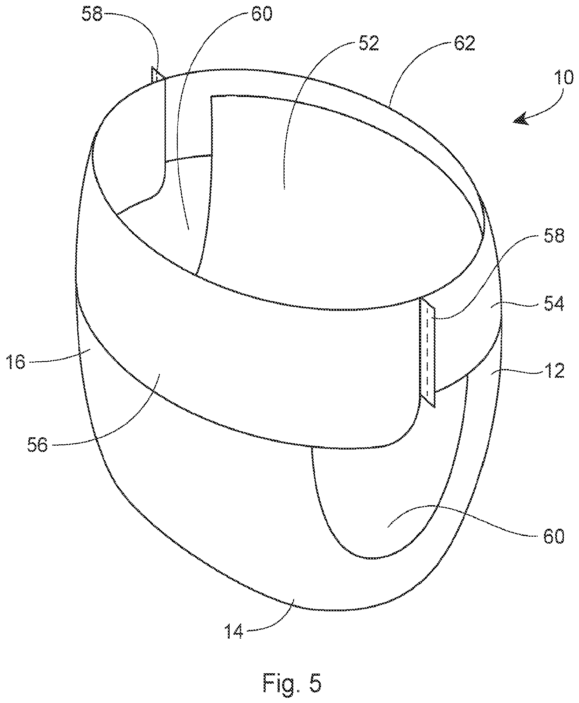

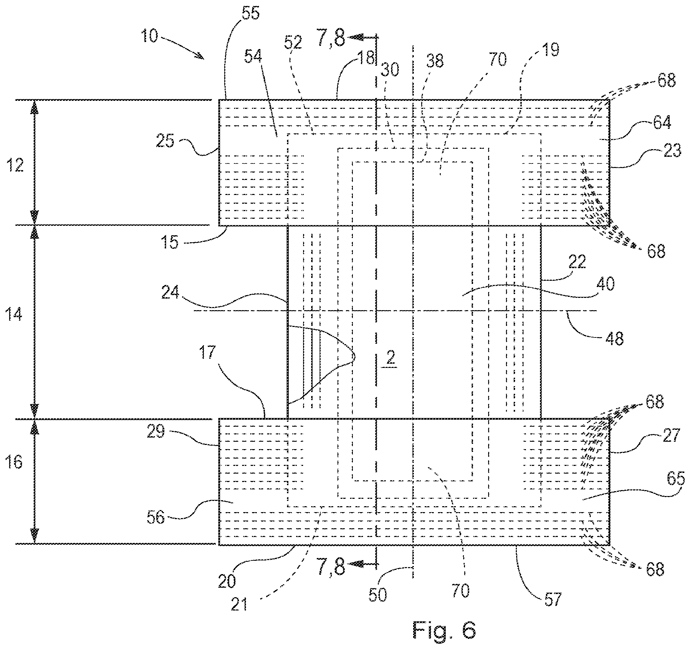

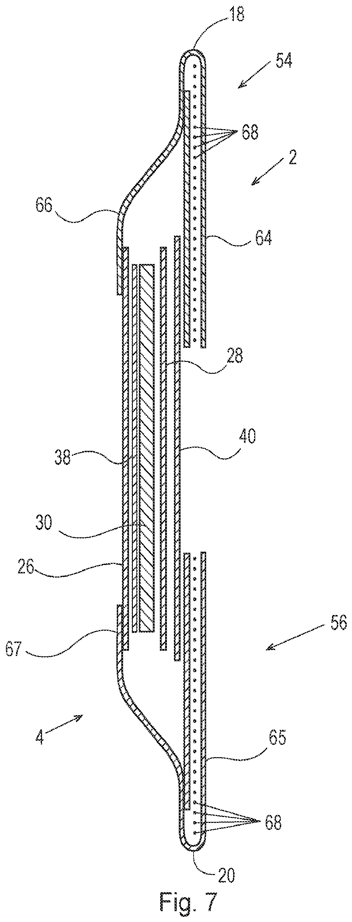

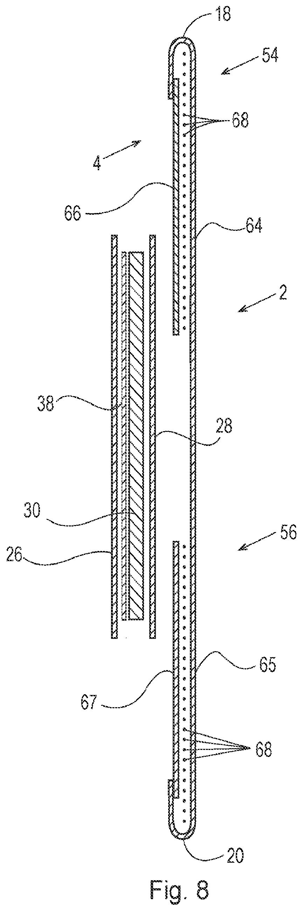

In other instances, the absorbent article may be in the form of a pant having permanent or refastenable side seams. Suitable refastenable seams are disclosed in U.S. Pat. Appl. Pub. No. 2014/0005020 and U.S. Pat. No. 9,421,137. Referring to Figs. 4-8, an example absorbent article 10 in the form of a pant is illustrated. Fig. 4 is a front perspective view of the absorbent article 10. Fig. 5 is a rear perspective view of the absorbent article 10. Fig. 6 is a plan view of the absorbent article 10, laid flat, with the garment-facing surface facing the viewer. Elements of Fig. 4-8 having the same reference number as described above with respect to Figs. 1-3 may be the same element (e.g., absorbent core 30). Fig. 7 is an example cross-sectional view of the absorbent article taken about line 7-7 of Fig. 6. Fig. 8 is an example cross-sectional view of the absorbent article taken about line 8-8 of Fig. 6. Figs. 7 and 8 illustrate example forms of front and back belts 54, 56. The absorbent article 10 may have a front waist region 12, a crotch region 14, and a back waist region 16. Each of the regions 12, 14, and 16 may be 1/3 of the length of the absorbent article 10. The absorbent article 10 may have a chassis 52 (sometimes referred to as a central chassis or central panel) comprising a topsheet 26, a backsheet 28, and an absorbent core 30 disposed at least partially intermediate the topsheet 26 and the backsheet 28, and an optional acquisition material 38, similar to that as described above with respect to Figs. 1-3. The absorbent article 10 may comprise a front belt 54 in the front waist region 12 and a back belt 56 in the back waist region 16. The chassis 52 may be joined to a wearer-facing surface 4 of the front and back belts 54, 56 or to a garment-facing surface 2 of the belts 54, 56. Side edges 23 and 25 of the front belt 54 may be joined to side edges 27 and 29, respectively, of the back belt 56 to form two side seams 58. The side seams 58 may be any suitable seams known to those of skill in the art, such as butt seams or overlap seams, for example. When the side seams 58 are permanently formed or refastenably closed, the absorbent article 10 in the form of a pant has two leg openings 60 and a waist opening circumference 62. The side seams 58 may be permanently joined using adhesives or bonds, for example, or may be refastenably closed using hook and loop fasteners, for example.

### Belts

Referring to Figs. 7 and 8, the front and back belts 54 and 56 may comprise front and back inner belt layers 66 and 67 and front and back outer belt layers 64 and 65 having an elastomeric material (e.g., strands 68 or a film (which may be apertured)) disposed at least partially therebetween. The elastic elements 68 or the film may be relaxed (including being cut) to reduce elastic strain over the absorbent core 30 or, may alternatively, run continuously across the absorbent core 30. The elastics elements 68 may have uniform or variable spacing therebetween in any portion of the belts. The elastic elements 68 may also be pre-strained the same amount or different amounts. The front and/or back belts 54 and 56 may have one or more elastic element free zones 70 where the chassis 52 overlaps the belts 54, 56. In other instances, at least some of the elastic elements 68 may extend continuously across the chassis 52.

The front and back inner belt layers 66, 67 and the front and back outer belt layers 64, 65 may be joined using adhesives, heat bonds, pressure bonds or thermoplastic bonds. Various suitable belt layer configurations can be found in U.S. Pat. Appl. Pub. No. 2013/0211363.

Front and back belt end edges 55 and 57 may extend longitudinally beyond the front and back chassis end edges 19 and 21 (as shown in Fig. 6) or they may be co-terminus. The front and back belt side edges 23, 25, 27, and 29 may extend laterally beyond the chassis side edges 22 and 24. The front and back belts 54 and 56 may be continuous (i.e., having at least one layer that is continuous) from belt side edge to belt side edge (e.g., the transverse distances from 23 to 25 and from 27 to 29). Alternatively, the front and back belts 54 and 56 may be discontinuous from belt side edge to belt side edge (e.g., the transverse distances from 23 to 25 and 27 to 29), such that they are discrete.

As disclosed in U.S. Pat. No. 7,901,393, the longitudinal length (along the central longitudinal axis 50) of the back belt 56 may be greater than the longitudinal length of the front belt 54, and this may be particularly useful for increased buttocks coverage when the back belt 56 has a greater longitudinal length versus the front belt 54 adjacent to or immediately adjacent to the side seams 58.

The front outer belt layer 64 and the back outer belt layer 65 may be separated from each other, such that the layers are discrete or, alternatively, these layers may be continuous, such that a layer runs continuously from the front belt end edge 55 to the back belt end edge 57. This may also be true for the front and back inner belt layers 66 and 67 - that is, they may also be longitudinally discrete or continuous. Further, the front and back outer belt layers 64 and 65 may be longitudinally continuous while the front and back inner belt layers 66 and 67 are longitudinally discrete, such that a gap is formed between them - a gap between the front and back inner and outer belt layers 64, 65, 66, and 67 is shown in Fig. 7 and a gap between the front and back inner belt layers 66 and 67 is shown in Fig. 8.

The front and back belts 54 and 56 may include slits, holes, and/or perforations providing increased breathability, softness, and a garment-like texture. Underwear-like appearance can be enhanced by substantially aligning the waist and leg edges at the side seams 58 (see Figs. 4 and 5).

The front and back belts 54 and 56 may comprise graphics (see e.g., 78 of Fig. 1). The graphics may extend substantially around the entire circumference of the absorbent article 10 and may be disposed across side seams 58 and/or across proximal front and back belt seams 15 and 17; or, alternatively, adjacent to the seams 58, 15, and 17 in the manner described in U.S. Pat. No. 9,498, 389 to create a more underwear-like article. The graphics may also be discontinuous. The graphics may be formed in whole or in part by any ink composition disclosed herein.

Alternatively, instead of attaching belts 54 and 56 to the chassis 52 to form a pant, discrete side panels may be attached to side edges of the chassis 22 and 24. Suitable forms of pants comprising discrete side panels are disclosed in U.S. Pat. Nos. 6,645,190; 8,747,379; 8,372,052; 8,361,048; 6,761,711; 6,817,994; 8,007,485; 7,862,550; 6,969,377; 7,497,851; 6,849,067; 6,893,426; 6,953,452; 6,840,928; 8,579,876; 7,682,349; 7,156,833; and 7,201,744.

### Topsheet

The topsheet 26 is the part of the absorbent article 10 that is in contact with the wearer's skin. The topsheet 26 may be joined to portions of the backsheet 28, the absorbent core 30, the barrier leg cuffs 32, and/or any other layers as is known to those of ordinary skill in the art. The topsheet 26 may be compliant, soft-feeling, and non-irritating to the wearer's skin. Further, at least a portion of, or all of, the topsheet may be liquid permeable, permitting liquid bodily exudates to readily penetrate through its thickness. A suitable topsheet may be manufactured from a wide range of materials, such as porous foams, reticulated foams, apertured plastic films, woven materials, nonwoven materials, woven or nonwoven materials of natural fibers (e.g., wood or cotton fibers), synthetic fibers or filaments (e.g., polyester or polypropylene or bicomponent PE/PP fibers or mixtures thereof), or a combination of natural and synthetic fibers. The topsheet may have one or more layers. The topsheet may be apertured (Fig. 2, element 31), may have any suitable three-dimensional features, and/or may have a plurality of embossments (e.g., a bond pattern). The topsheet may be apertured by overbonding a material and then rupturing the overbonds through ring rolling, such as disclosed in U.S. Patent No. 5,628,097, to Benson et al., issued on May 13, 1997 and disclosed in U.S. Pat. Appl. Publication No. US 2016/0136014 to Arora et al. Any portion of the topsheet may be coated with a skin care composition, an antibacterial agent, a surfactant, and/or other beneficial agents. The topsheet may be hydrophilic or hydrophobic or may have hydrophilic and/or hydrophobic portions or layers. If the topsheet is hydrophobic, typically apertures will be present so that bodily exudates may pass through the topsheet.

### Backsheet

The backsheet 28 is generally that portion of the absorbent article 10 positioned proximate to the garment-facing surface of the absorbent core 30. The backsheet 28 may be joined to portions of the topsheet 26, the outer cover material 40, the absorbent core 30, and/or any other layers of the absorbent article by any attachment methods known to those of skill in the art. The backsheet 28 prevents, or at least inhibits, the bodily exudates absorbed and contained in the absorbent core 10 from soiling articles such as bedsheets, undergarments, and/or clothing. The backsheet is typically liquid impermeable, or at least substantially liquid impermeable. The backsheet may, for example, be or comprise a thin plastic film, such as a thermoplastic film having a thickness of about 0.012 mm to about 0.051 mm. Other suitable backsheet materials may include breathable materials which permit vapors to escape from the absorbent article, while still preventing, or at least inhibiting, bodily exudates from passing through the backsheet.

### Outer Cover Material

The outer cover material (sometimes referred to as a backsheet nonwoven) 40 may comprise one or more nonwoven materials joined to the backsheet 28 and that covers the backsheet 28. The outer cover material 40 forms at least a portion of the garment-facing surface 2 of the absorbent article 10 and effectively "covers" the backsheet 28 so that film is not present on the garment-facing surface 2. The outer cover material 40 may comprise a bond pattern, apertures, and/or three-dimensional features. The outer cover material 40 may be a hydroentangled nonwoven material.

### Absorbent Core

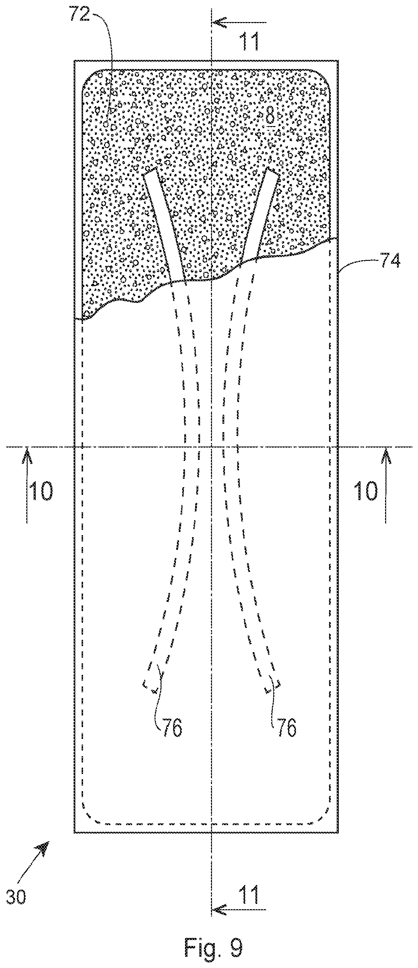

As used herein, the term "absorbent core" 30 refers to a component of the absorbent article 10 disposed in the article for absorbing and containing liquid such as urine received by the absorbent article. The absorbent core thus typically has a high absorbent capacity. An example absorbent core 30 is schematically shown in Figs. 9-11. The absorbent core comprises an absorbent material 72, that is typically enclosed within or sandwiched between a core wrap 74.

The core wrap may be a single material that is folded and attached to itself, or it may comprise a separate top layer and bottom layer that may be bonded or otherwise joined together. The absorbent material typically comprises superabsorbent particles which are optionally mixed with cellulose fibers. As used herein, "absorbent core" does not include any acquisition-distribution systems, topsheet, or backsheet of the absorbent article.

The example absorbent core 30 shown in isolation in Figs. 9-11 is in the dry state (before use). The absorbent core may typically have a generally rectangular shape as defined by its longitudinal edges and transversal front edge and back edge or may have other shapes.

Absorbent material 72 may be deposited as an absorbent layer having a generally rectangular outline, as represented in Fig. 9. A wide variety of absorbent cores may also be used. The absorbent material 72 layer may also have a non-rectangular perimeter ("shaped" core), in particular, the absorbent material 72 may define a tapering along its width towards the central region of the core (or "dog-bone" shape). In this way, the absorbent material deposition area may have a relatively narrow width in an area of the core intended to be placed in the crotch region of the absorbent article. This may provide for example better wearing comfort. Other shapes can also be used such as a "T" or "Y" or "sand-hour" for the area of the absorbent material.

The absorbent material 72 may be any conventional absorbent material known in the art. For example, the absorbent material may comprise a blend of cellulose fibers and superabsorbent particles ("SAP"), typically with the percentage of SAP ranging from about 50% to about 75% by weight of the absorbent material. The absorbent material may also be free of cellulose fibers, as is known in so-called airfelt-free cores, where the absorbent material consists, or consists essentially, of SAP. The absorbent material may also be a high internal phase emulsion foam
"Superabsorbent polymer" or "SAP" refers herein to absorbent materials, typically cross-linked polymeric materials, that can absorb at least 10 times their weight of an aqueous 0.9% saline solution as measured using the Centrifuge Retention Capacity (CRC) test (EDANA method WSP 241.2.R3 (12)). The SAP may in particular have a CRC value of at least 20 g/g, in particular of from 20 g/g to 40 g/g. "Superabsorbent polymer particles", as used herein, refers to a superabsorbent polymer material which is in particulate form so as to be flowable in the dry state.

Various absorbent core designs comprising high amounts of SAP have been proposed in the past, see for example in U.S. Pat. No. 5,599,335 (Goldman), EP 1447066 (Busam), WO 95/11652 (Tanzer), U.S. Pat. Appl. Pub. No. 2008/0312622 A1 (Hundorf), WO 2012/052172 (Van Malderen). In particular, the SAP printing technology as disclosed in U.S. Pat. Appl. Pub. No. 2006/024433 (Blessing), U.S. Pat. Appl. Pub. No. 2008/0312617 and U.S. Pat. Appl. Pub. No. 2010/0051166 A1 (both to Hundorf et al.) may be used. The present disclosure however is not limited to a particular type of absorbent core. The absorbent core may also comprise one or more glues such as an auxiliary glue applied between the internal surface of one (or both) of the core wrap layers and the absorbent material to reduce leakage of SAP outside the core wrap. A microfibrous adhesive net may also be used in air-felt free cores as described in the above Hundorf references. These glues are not represented in the Figures for simplicity. Other core constructions comprising a high loft nonwoven substrate such as a carded nonwoven layer, having a porous structure into which SAP particles have been deposited, may also be used in present disclosure.

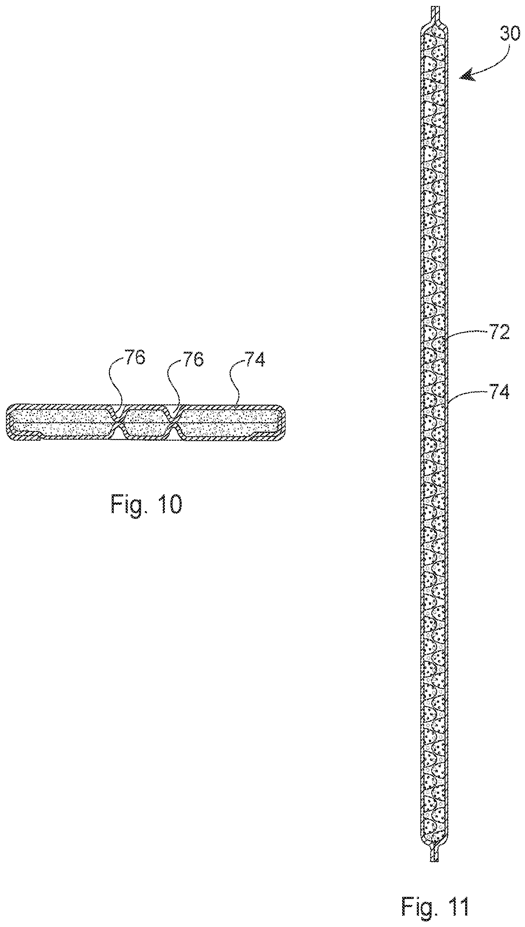

The absorbent material may be deposited as a continuous layer within the core wrap. The absorbent material may also be present discontinuously, for example, as individual pockets or stripes of absorbent material enclosed within the core wrap and separated from each other by material-free junction areas. A continuous layer of absorbent material, in particular of SAP, may also be obtained by combining two absorbent layers having matching discontinuous absorbent material application pattern, wherein the resulting layer is substantially continuously distributed across the absorbent particulate polymer material area, as illustrated in Figs. 10-11. As for example taught in U.S. Pat. Appl. Pub. No. 2008/312622 A1 (Hundorf), each absorbent material layer may thus comprise a pattern having absorbent material land areas and absorbent material-free junction areas, wherein the absorbent material land areas of the first layer correspond substantially to the absorbent material-free junction areas of the second layer and vice versa.

The basis weight (amount deposited per unit of surface) of the absorbent material may also be varied to create a profiled distribution of absorbent material, in particular in the longitudinal direction to provide more absorbency towards the center and the middle of the core, but also in the transversal direction, or both directions of the core. The absorbent core may also comprise one or more longitudinally (or otherwise) extending channels 76, which are areas of the absorbent layer substantially free of absorbent material within the absorbent material layer. The top side of the core wrap may be advantageously bonded to the bottom side of the core by adhesive, mechanical or ultra-sonic bonding through these material-free areas. Example disclosures of such channels in an airfelt-free core can be found in WO 2012/170778 (Rosati et al.) and US 2012/0312491 (Jackels). Channels may of course also be formed in absorbent cores comprising a mix of cellulose fibers and SAP particles. These channels may embody any suitable shapes and any suitable number of channels may be provided. In other instances, the absorbent core may be embossed to create the impression of channels. The absorbent core in Figs. 9-11 is merely an example absorbent core. Many other absorbent cores with or without channels are also within the scope of the present disclosure.

### Barrier Leg Cuffs/Leg Elastics

Referring to Figs. 1 and 2, for example, the absorbent article 10 may comprise one or more pairs of barrier leg cuffs 32 and one or more pairs of leg elastics 34. The barrier leg cuffs 32 may be positioned laterally inboard of leg elastics 34. Each barrier leg cuff 32 may be formed by a piece of material which is bonded to the absorbent article 10 so it can extend upwards from a wearer-facing surface 4 of the absorbent article 10 and provide improved containment of body exudates approximately at the junction of the torso and legs of the wearer. The barrier leg cuffs 32 are delimited by a proximal edge joined directly or indirectly to the topsheet and/or the backsheet and a free terminal edge, which is intended to contact and form a seal with the wearer's skin. The barrier leg cuffs 32 may extend at least partially between the front end edge 18 and the back end edge 20 of the absorbent article 10 on opposite sides of the central longitudinal axis 50 and may be at least present in the crotch region 14. The barrier leg cuffs 32 may each comprise one or more elastics 33 (e.g., elastic strands or strips) near or at the free terminal edge. These elastics 33 cause the barrier leg cuffs 32 to help form a seal around the legs and torso of a wearer. The leg elastics 34 extend at least partially between the front end edge 18 and the back end edge 20. The leg elastics 34 essentially cause portions of the absorbent article 10 proximate to the chassis side edges 22, 24 to help form a seal around the legs of the wearer. The leg elastics 34 may extend at least within the crotch region 14.

### Elastic Waistband

Referring to Figs. 1 and 2, the absorbent article 10 may comprise one or more elastic waistbands 36. The elastic waistbands 36 may be positioned on the garment-facing surface 2 or the wearer-facing surface 4. As an example, a first elastic waistband 36 may be present in the front waist region 12 near the front belt end edge 18 and a second elastic waistband 36 may be present in the back waist region 16 near the back end edge 20. The elastic waistbands 36 may aid in sealing the absorbent article 10 around a waist of a wearer and at least inhibiting bodily exudates from escaping the absorbent article 10 through the waist opening circumference. In some instances, an elastic waistband may fully surround the waist opening circumference of an absorbent article.

### Acquisition Materials

Referring to Figs. 1, 2, 7, and 8, one or more acquisition materials 38 may be present at least partially intermediate the topsheet 26 and the absorbent core 30. The acquisition materials 38 are typically hydrophilic materials that provide significant wicking of bodily exudates. These materials may dewater the topsheet 26 and quickly move bodily exudates into the absorbent core 30. The acquisition materials 38 may comprise one or more nonwoven materials, foams, cellulosic materials, cross-linked cellulosic materials, air laid cellulosic nonwoven materials, spunlace materials, or combinations thereof, for example. In some instances, portions of the acquisition materials 38 may extend through portions of the topsheet 26, portions of the topsheet 26 may extend through portions of the acquisition materials 38, and/or the topsheet 26 may be nested with the acquisition materials 38. Typically, an acquisition material 38 may have a width and length that are smaller than the width and length of the topsheet 26. The acquisition material may be a secondary topsheet in the feminine pad context. The acquisition material may have one or more channels as described above with reference to the absorbent core 30 (including the embossed version). The channels in the acquisition material may align or not align with channels in the absorbent core 30. In an example, a first acquisition material may comprise a nonwoven material and as second acquisition material may comprise a cross-linked cellulosic material.

### Landing Zone

Referring to Figs. 1 and 2, the absorbent article 10 may have a landing zone area 44 that is formed in a portion of the garment-facing surface 2 of the outer cover material 40. The landing zone area 44 may be in the back waist region 16 if the absorbent article 10 fastens from front to back or may be in the front waist region 12 if the absorbent article 10 fastens back to front. In some instances, the landing zone 44 may be or may comprise one or more discrete nonwoven materials that are attached to a portion of the outer cover material 40 in the front waist region 12 or the back waist region 16 depending upon whether the absorbent article fastens in the front or the back. In essence, the landing zone 44 is configured to receive the fasteners 46 and may comprise, for example, a plurality of loops configured to be engaged with, a plurality of hooks on the fasteners 46, or *vice versa.*

### Wetness Indicator/Graphics

Referring to Fig. 1, the absorbent articles 10 of the present disclosure may comprise graphics 78 and/or wetness indicators 80 that are visible from the garment-facing surface 2. The graphics 78 may be printed on the landing zone 40, the backsheet 28, and/or at other locations. The graphics 78 may be formed in whole or in part by any ink composition disclosed herein. The wetness indicators 80 are typically applied to the absorbent core facing side of the backsheet 28, so that they can be contacted by bodily exudates within the absorbent core 30. In some instances, the wetness indicators 80 may form portions of the graphics 78. For example, a wetness indicator may appear or disappear and create/remove a character within some graphics. In other instances, the wetness indicators 80 may coordinate (e.g., same design, same pattern, same color) or not coordinate with the graphics 78.

### Front and Back Ears

Referring to Figs. 1 and 2, as referenced above, the absorbent article 10 may have front and/or back ears 47, 42 in a taped diaper context. Only one set of ears may be required in most taped diapers. The single set of ears may comprise fasteners 46 configured to engage the landing zone or landing zone area 44. If two sets of ears are provided, in most instances, only one set of the ears may have fasteners 46, with the other set being free of fasteners. The ears, or portions thereof, may be elastic or may have elastic panels. In an example, an elastic film or elastic strands may be positioned intermediate a first nonwoven material and a second nonwoven material. The elastic film may or may not be apertured. The ears may be shaped. The ears may be integral (e.g., extension of the outer cover material 40, the backsheet 28, and/or the topsheet 26) or may be discrete components attached to a chassis 52 of the absorbent article on a wearer-facing surface 4, on the garment-facing surface 2, or intermediate the two surfaces 4, 2.

### Masking Layer

One or more masking layers or materials may be provided in the absorbent articles 10. A masking layer may be a layer that provides a cushiony feel when the absorbent article is touched from the garment-facing surface 2 or the wearer-facing surface 4. The masking layer may "mask" a grainy feel potentially caused by the absorbent material 72, such as superabsorbent polymers. The masking layer may "mask" bodily exudates from being visible when viewing the wearer-facing surface 4 or the garment-facing surface 2 of the absorbent article 10. The masking layer may have a basis weight in the range of about 15 gsm to about 50 gsm or about 15 gsm to about 40 gsm. The masking layer may comprise one or more nonwoven materials (e.g., a hydroentangled nonwoven material), foams, pulp layers, and/or other suitable materials. The masking layer may be the outer cover material 40. The masking layer may be the layer forming the garment-facing side or the wearer-facing side of the core bag 74. The masking layer may be a separate material positioned intermediate the garment-facing side of the core bag 74 and the liquid impermeable backsheet 28.

### Sensors

Referring again to Fig. 1, the absorbent articles of the present disclosure may comprise a sensor system 82 for monitoring changes within the absorbent article 10. The sensor system 82 may be discrete from or integral with the absorbent article 10. The absorbent article 10 may comprise sensors that can sense various aspects of the absorbent article 10 associated with insults of bodily exudates such as urine and/or BM (e.g., the sensor system 82 may sense variations in temperature, humidity, presence of ammonia or urea, various vapor components of the exudates (urine and feces), changes in moisture vapor transmission through the absorbent articles garment-facing layer, changes in translucence of the garment-facing layer, and/or color changes through the garment-facing layer). Additionally, the sensor system 82 may sense components of urine, such as ammonia or urea and/or byproducts resulting from reactions of these components with the absorbent article 10. The sensor system 82 may sense byproducts that are produced when urine mixes with other components of the absorbent article 10 (e.g., adhesives, agm). The components or byproducts being sensed may be present as vapors that may pass through the garment-facing layer. It may also be desirable to place reactants in the absorbent article that change state (e.g. color, temperature) or create a measurable byproduct when mixed with urine or BM. The sensor system 82 may also sense changes in pH, pressure, odor, the presence of gas, blood, a chemical marker or a biological marker or combinations thereof. The sensor system 82 may have a component on or proximate to the absorbent article that transmits a signal to a receiver more distal from the absorbent article, such as an iPhone, for example. The receiver may output a result to communicate to the caregiver a condition of the absorbent article 10. In other instances, a receiver may not be provided, but instead the condition of the absorbent article 10 may be visually or audibly apparent from the sensor on the absorbent article.

### Sanitary Napkin

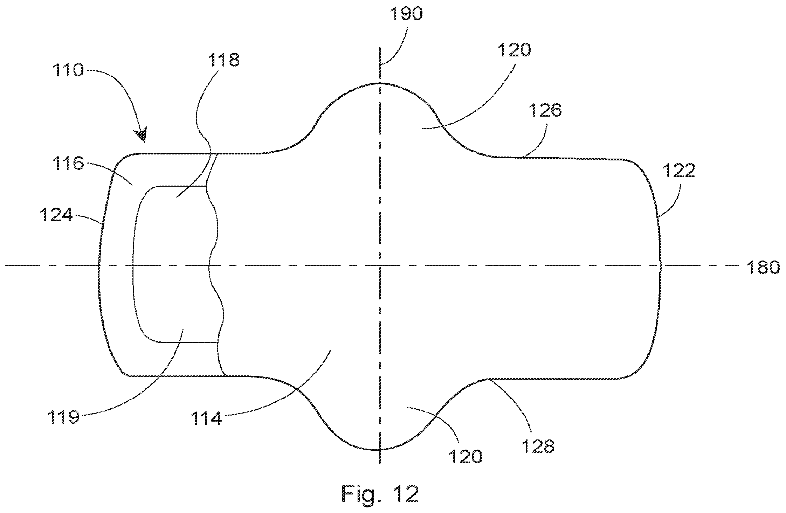

Referring to Fig. 12, an absorbent article of the present disclosure may be a sanitary napkin 110. The sanitary napkin 110 may comprise a liquid permeable topsheet 114, a liquid impermeable, or substantially liquid impermeable, backsheet 116, and an absorbent core 118. The liquid impermeable backsheet 116 may or may not be vapor permeable. The absorbent core 118 may have any or all of the features described herein with respect to the absorbent core 30 and, in some forms, may have a secondary topsheet 119 (STS) instead of the acquisition materials disclosed above. The STS 119 may comprise one or more channels, as described above (including the embossed version). In some forms, channels in the STS 119 may be aligned with channels in the absorbent core 118. The sanitary napkin 110 may also comprise wings 120 extending outwardly with respect to a longitudinal axis 180 of the sanitary napkin 110. The sanitary napkin 110 may also comprise a lateral axis 190. The wings 120 may be joined to the topsheet 114, the backsheet 116, and/or the absorbent core 118. The sanitary napkin 110 may also comprise a front edge 122, a back edge 124 longitudinally opposing the front edge 122, a first side edge 126, and a second side edge 128 longitudinally opposing the first side edge 126. The longitudinal axis 180 may extend from a midpoint of the front edge 122 to a midpoint of the back edge 124. The lateral axis 190 may extend from a midpoint of the first side edge 128 to a midpoint of the second side edge 128. The sanitary napkin 110 may also be provided with additional features commonly found in sanitary napkins as is known in the art.

### Perfume Compositions

Absorbent articles of the present disclosure may comprise a perfume composition. The perfume composition may comprise one or more perfume components and a carrier component.

The perfume may comprise a perfume component selected from the group of: hexyl cinnamic aldehyde, alpha-amylcinnamic aldehyde, p-anisaldehyde, benzaldehyde, cinnamic aldehyde, cuminic aldehyde, decanal, cyclamen aldehyde, beta-ionone, p-t-butyl-alpha-methyldihydrocinnamaldehyde, 3-methyl-5-phenyl-1-pentanol (phenyl hexanol), 4-hydroxy-3-methoxycinnamaldehyde, vanillin isobutyrate, 2-phenyl-3-(2-furyl)prop-2-enal, ethyl vanillin acetate, vanillin acetate, heptanal, lauryl aldehyde, nonanal, octanal, phenylacetaldehyde, phenyl propyl aldehyde, vanillin, salycil aldehyde, cytral, 2,4-dihydroxy-3-methylbenzaldehyde, 2-hydroxy-4-methylbenzaldehyde, 5-methyl salicylic aldehydes, 4-nitrobenzaldehyde, o-nitrobenzaldehyde, 5-ethyl-2-thiophenecarbaldehyde, 5-methyl-2-thiophenecarboxaldehyde, 2-thiophenecarbaldehyde, asaronaldehyde, 5-(hydroxymethyl)-2-furaldehyde, 2-benzofurancarboxaldehyde, 2,3,4-trimethoxybenzaldehyde, protocatechualdehyde, heliotropine, 4-ethoxy-3-methoxy benzaldehyde, 3,4,5-trimethoxybenzaldehyde, 3-hydroxybenzaldehyde, o-methoxycinnamaldehyde, 3,5-dimethoxy-4-hydroxycinnamaldehyde, 2,8-dithianon-4-3n-4-carboxaldehyde, sorbinaldehyde, 2,4-heptadienal, 2,4-decadienal, 2,4-nonadienal, 2,4-nonadienal, (E,E)- ,2,4-octadien-1-al, 2,4-octadienal, 2,4-dodecadienal, 2,4-undecadienal, 2,4-tridecadien-1-al, 2-trans-4-cis-7-cis-tridecatrienal, piperonylidene propionaldehyde, 2-methyl-3-(2-furyl)acrolein, 2,4-pentadienal, 2-furfurylidene butyraldehyde, 3-(2-furyl)acrolein, pyruvaldehyde, ethanedial, Laevo-Carvone, 1-(2,6,6-trimethyl-1-cyclohexenyl)pent-1-en-3-one, 4-(2,6,6-trimethyl-1-cyclohexen-1-yl)-3-Buten-2-one, 4-(2,6,6-trimethyl-2-cyclohexen-1-yl)-3-buten-2-one (isomers), 5-(2,6,6-Trimethyl-2-cyclohexen-1-yl) 4-penten-3-one, (E)-4-(2,2-dimethyl-6-methylidenecyclohexyl)but-3-en-2-one, and mixtures thereof. Alternatively or in addition, the perfume composition may comprise one or more perfume components selected from the group of: menthol, menthyl acetate, menthyl lactate, 1-(2,6,6-trimethyl-1-cyclohexenyl)pent-1-en-3-one, 4-(2,6,6-trimethyl-1-cyclohexen-1-yl)-3-Buten-2-one, 4-(2,6,6-trimethyl-2-cyclohexen-1-yl)-3-buten-2-one (isomers), 5-(2,6,6-Trimethyl-2-cyclohexen-1-yl) 4-penten-3-one, (E)-4-(2,2-dimethyl-6-methylidenecyclohexyl)but-3-en-2-one, isomenthyl acetate, isomenthyl propionate, isomenthyl isobutyrate, isomenthyl propionate, isomenthyl butyrate, camphor, p-menthane, limonene, eucalyptol, cresol, linalool, tetra-hydrolinalool, myrcenol, tetra hydromyrcenol, di-hydromyrcenol, myrcene, cytronellol, cytronellyil derivatives, geraniol, geranyl derivatives, linalyl acetate, mugetanol, eugenol, jasmal, terpineol, pinanol, cedrene, damascone, beta pinene, cineole and its derivatives, nonadienol, ethylhexanal, octanol acetate, methyl furfural, terpinene, thujene, amylacetate, benzylacetate, camphene, citronellal, dihydrocumarin, dy hydromyrcenyl acetate, geraniol, geranial, isoamylacetate, ethyl, and/or triethyl acetate, para-cresol, para-cymene, methyl abietate, methyl dihydro jasmonate, hexyl-2-methyl butyrate, benzyl acetate, laevo carvone , hexyl-2-methyl butyrate, eucalyptus, phenyl ethyl alcohol, and mixtures thereof. Alternatively or in addition, the perfume composition may comprise limonene, eucalyptol, cresol, linalool, tetra-hydrolinalool, myrcenol, tetra hydromyrcenol, di-hydromyrcenol, myrcene, cytronellol, cytronellyil derivatives, geraniol, geranyl derivatives, linalyl acetate, mugetanol, eugenol, jasmal, terpineol, pinanol, cedrene, damascone, beta pinene, cineole and its derivatives, nonadienol, ethylhexanal, octanol acetate, methyl furfural, terpinene, thujene, amylacetate, benzylacetate, camphene, citronellal, di-hydrocumarin, di-hydromyrcenyl acetate, geraniol, geranial, encalyptus, isoamylacetate, ethyl, and /or triethyl acetate, para-cresol and para-cymene, benzyl-benzoate, methyl abietate, ethanol, isopropanol, diethylene glycol monoethyl ether, glycerol, propylene glycol, 1,2-butylene glycol, 2-methyl-2,4-pentanediol, diethyl phthalate, triethyl citrate, diethyl sebacate.

The perfume composition comprises between about 15 wt.% and about 85 wt.% of a carrier component. The carrier component may be a single component, or may be a blend of two or more components. The carrier may function to dilute the perfume component or components. The carrier may also, or alternatively, function to moderate the volatilization of the perfume composition by, for example, slowing evaporation and thereby creating a longer-lasting scent. The carrier component may be substantially odorless. The carrier component may be insoluble and/or immiscible in water. As used herein, the term "insoluble" means a component has a solubility in water of less than 1.0 mg/L at 20°C. As used herein, the term "soluble" means a component has a solubility in water of, or greater than, 1.0 mg/L at 20°C. The carrier component may be non-hygroscopic. The carrier component may have a low vapor pressure. The carrier component may have a vapor pressure of less than about 7.5x10⁻⁴ mmHg, or between about 7.5x10⁻⁴ mmHg and about 3.46x10⁻⁸ mmHg, between about 1.5x10⁻⁴ mmHg and about 5.0x10⁻⁷ mmHg, or between about 9.8x10⁻⁵ mmHg and about 7.5x10⁻⁶ mmHg, specifically reciting every value within these ranges and any ranges formed therein or thereby. The carrier component may have a partition coefficient (log K_{ow}) of greater than 1.0, between about 1.0 and about 10, between about 1.5 and about 8.5, or between about 2.0 and about 8, specifically reciting every value within these ranges and any ranges formed therein or thereby. The carrier component comprises isopropyl myristate. The carrier component may consist of isopropyl myristate. Without wishing to be bound by theory, it is believed that carrier components as described herein may exhibit limited or no ability to cross a packaging material and/or have limited or no ability to interact with ink and/or OPV disposed on an absorbent article and/or a packaging material.

The perfume composition of the present disclosure may comprise a perfume component having a partition coefficient (log K_{ow}) of less than 6, less than 4, less than 2, less than 1.5, between about 0.2 and about 6, between about 0.4 and about 4, between about 0.6 and about 2, or between about 0.75 and about 1.5, specifically reciting every value within these ranges and any ranges formed therein or thereby in combination with a carrier component as discussed above. The perfume composition of the present disclosure may comprise a perfume component having a partition coefficient (log K_{ow}) of less than 6 and a carrier component that is insoluble in water and/or the carrier component having a vapor pressure of less than about 7.5x10⁻⁴ mmHg and/or the carrier component having a partition coefficient (log K_{ow}) of between about 1.0 and about 10. In a form, the perfume composition of the present disclosure may comprise a perfume component having a partition coefficient (log K_{ow}) of between about 0.2 and about 6 and a carrier component having a vapor pressure of between about 7.5x10⁻⁴ mmHg and about 3.46x10⁻⁸ mmHg. In another form, the perfume composition of the present disclosure may comprise a perfume component having a partition coefficient (log K_{ow}) of between about 0.4 and about 4 and a carrier component having a vapor pressure of between about 1.5x10⁻⁴ mmHg and about 5.0x10⁻⁷ mmHg and/or a partition coefficient (log K_{ow}) between about 2.0 and about 8. Such formulations of the present disclosure are believed to have limited or no ability to diffuse through a packaging material and/or have limited or no ability to interact with ink and/or OPV disposed on an absorbent article and/or a packaging material.

The perfume composition may be applied in a variety of ways, and in a variety of patterns, to the absorbent article using fluid application equipment such as spray, droplets, or beads applicators. Such applicators allow application of the perfume composition to the absorbent article in any pattern, such as stripes, circles, dots, drops, geometric figures, stars, decorative figures, irregular shapes, and the like. Patterned applications may be beneficial because they allow a precise application so that it is easier to avoid contact with the glue which connects the various layers of the absorbent article.

The perfume composition may be applied to the finished absorbent article before the absorbent article is placed into a package. The perfume composition may be applied to a component or multiple components of the absorbent article during the process of assembling the absorbent article. For example, the perfume composition may be applied on or within a layer of the absorbent article. This means that, since the absorbent article is constituted by a series of layers, the perfume composition may be applied onto one of the surfaces of these layers. For example, the perfume composition may be applied to a surface of the core wrap and/or the acquisition layer and/or the secondary topsheet and/or the topsheet. Alternatively, if one of the layers allows it (because for example is a thick fibrous layer such as an absorbent core), the layer may be cut in two along a plane substantially parallel to the garment facing surface of the article and the perfume composition may be applied on one of the two surfaces resulting from the cut and then the layer may be re-joined as a single layer. The perfume composition may be applied to a component material of the absorbent article before the component material is engaged with other component materials to form the absorbent article.

The perfume composition may be disposed on the absorbent article an/or on a component or components of the absorbent article in an amount of from about 1 mg to about 500 mg per absorbent article, from about 3 mg to about 200 mg per absorbent article or from about 4 mg to about 150 mg per absorbent article, specifically reciting every value within these ranges and any ranges formed therein or thereby.

### Bio-Based Content for Components

Components of the absorbent articles and/or packaging materials described herein may at least partially be comprised of bio-based content as described in U.S. Pat. Appl. No. 2007/0219521 A1. For example, the superabsorbent polymer component may be bio-based via their derivation from bio-based acrylic acid. Bio-based acrylic acid and methods of production are further described in U.S. Pat. Appl. Pub. No. 2007/0219521 and U.S. Pat. Nos. 8,703,450; 9,630,901 and 9,822,197. Other components, for example nonwoven and film components, may comprise bio-based polyolefin materials. Bio-based polyolefins are further discussed in U.S. Pat. Appl. Pub. Nos. 2011/0139657, 2011/0139658, 2011/0152812, and 2016/0206774, and U.S. Pat. No. 9,169,366. Example bio-based polyolefins for use in the present disclosure comprise polymers available under the designations SHA7260^{™}, SHE150^{™}, or SGM9450F^{™} (all available from Braskem S.A.).

An absorbent article component may comprise a bio-based content value from about 10% to about 100%, from about 25% to about 100%, from about 40% to about 100%, from about 50% to about 100%, from about 75% to about 100%, or from about 90% to about 100%, for example, using ASTM D6866-10, method B.

### Recycle Friendly Absorbent Articles and Packaging Materials

Components of the absorbent articles and/or packaging materials described herein may be recycled for other uses, whether they are formed, at least in part, from recyclable materials. Examples of absorbent article materials that may be recycled are nonwovens, films, fluff pulp, and superabsorbent polymers. The recycling process may use an autoclave for sterilizing the absorbent articles, after which the absorbent articles may be shredded and separated into different byproduct streams. Example byproduct streams may comprise plastic, superabsorbent polymer, and cellulose fiber, such as pulp. These byproduct streams may be used in the production of fertilizers, plastic articles of manufacture, paper products, viscose, construction materials, absorbent pads for pets or on hospital beds, and/or for other uses. Further details regarding absorbent articles that aid in recycling, designs of recycle friendly diapers, and designs of recycle friendly and bio-based component diapers, are disclosed in U.S. Pat. Appl. Publ. No. 2019/0192723, published on June 27, 2019.

### Packages

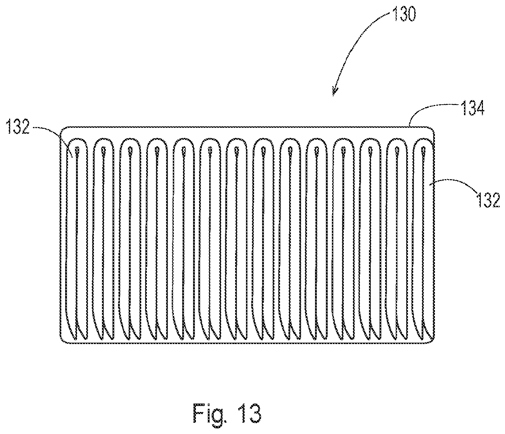

Referring to Fig. 13, absorbent articles 132 of the present disclosure may be placed into a package 130. Graphics and/or indicia may be formed on, printed on, positioned on, and/or placed on outer surfaces of the package 130. Each package 130 may comprise a plurality of absorbent articles 132. The absorbent articles 132 may be packed under compression so as to reduce the size of the package, while still providing an adequate number of absorbent articles per package. By packaging the absorbent articles under compression, caregivers can easily handle and store the packages, while also providing distribution savings to manufacturers owing to the size of the packages.

### Packaging Materials:

The packages 130 of the present disclosure may comprise packaging materials 134 comprising polymeric films and/or other materials. Polymeric films may comprise polyolefins, particularly polyethylenes, polypropylenes, polybutadienes, polypropylene-ethylene interpolymer and copolymers having at least one olefinic constituent, and any mixtures thereof. Polyolefins may include, but are not limited to, linear low density polyethylene (LLDPE), low density polyethylene (LDPE), medium density polyethylene (MDPE), high density polyethylene (HDPE), polymethylpentene ("PMP"), polybutene-1 ("PB-1"), isotactic polypropylene, random polypropylene copolymers, impact modified polypropylene copolymer, polyisobutene ("PIB"), crosslinked polyethylene (XLPE), crosslinked high density polyethylene (PEX), and other polyolefins which are described in PCT Application Nos. WO 99/20664, WO 2006/047374, and WO 2008/086539. Other base polymers such as polyesters, nylons, polyhydroxyalkanoates (or PHAs), polyvinyl alcohol, copolymers thereof, and combinations of any of the foregoing may also be suitable. In addition, polyolefin plastomers and elastomers may be utilized to form a multi-layer polymeric films. Examples of such suitable polyolefin plastomers and elastomers are described in U.S. Pat. No. 6,258,308; U.S. Publication No. 2010/0159167 A1; and PCT Application Nos. WO 2006/047374 and WO 2006/017518.

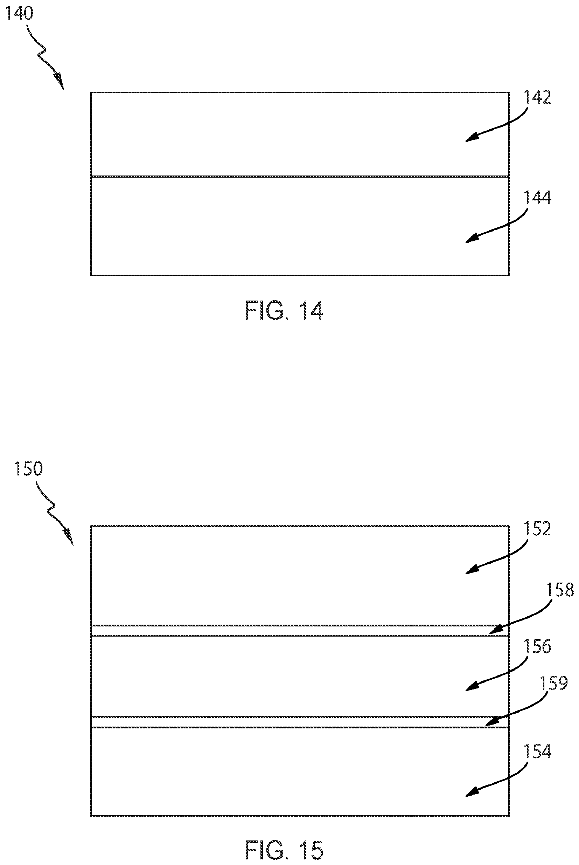

The polymeric film may be a single layer (monolayer), or may have two, three or more layers (multilayer). Referring to Fig. 14, multilayer film 140 may have, for example, an outer skin layer 142 formed of a first polymer and an inner skin layer 144 formed of a second polymer. (As used herein, the terms "outer" and "inner" refer to the positioning of the layer relative the inside and the outside of the finished package; thus, the "inner layer" faces the contained product, and the "outer layer" faces outward and has an outer surface that is exposed to view and touch by, *e.g.,* shoppers in a retail store.) A multilayer film may comprise one or more tie layers disposed between other layers. A tie layer may be beneficial when the polymers of adjoining layers would not otherwise be miscible or compatible so as to bond to each other during extrusion. For example, a tie layer between a polyethylene skin layer and an intermediate layer having a large polylactic acid content may be desirable because of the significant difference in polarity between polylactic acid (PLA) and polyolefins. Referring to Fig. 15, in a multilayer film 150 having three main layers - two skin layers (152, 154) and an intermediate layer 156 disposed between them, tie layers 158, 159 may be disposed between the intermediate layer 156 and each of the skin layers 152, 154. A tie layer may include one or more functionalized polyolefins. In some example, a tie layer may include from 5%, 10%, 20%, 30%, 40% or 45% to 55%, 60%, 70%, 80%, 90%, or 100%, by weight of the tie layer, of the one or more functionalized polyolefins. A tie layer may consist essentially of the one or more functionalized polyolefins.

The packages of the present disclosure may comprise packaging materials comprising natural fibers. The term "natural fibers" as used herein, refers to fibers which comprise cellulose-based fibers, bamboo fibers, and the like. Natural fibers also refers to: nonwoody fibers, such as cotton, abaca, kenaf, sabai grass, flax, esparto grass, straw, jute, hemp, bagasse, milkweed floss fibers, and pineapple leaf fibers; and woody fibers, such as wood or pulp fibers such as those obtained from deciduous and coniferous trees, including softwood fibers, such as northern and southern softwood kraft fibers, hardwood fibers, such as eucalyptus, maple, birch, and aspen. The packaging materials may comprise at least 50 percent by weight natural fibers, at least 70 percent by weight natural fibers, at least 90 percent by weight natural fibers, between about 50 percent and about 100 percent by weight natural fibers, between about 65 percent and about 99 percent by weight natural fibers, or between about 75 percent and about 95 percent by weight of natural fibers, specifically reciting all values within these ranges and any ranges formed therein or thereby. In one form, the package materials may comprise 99.9% percent by weight natural fibers.

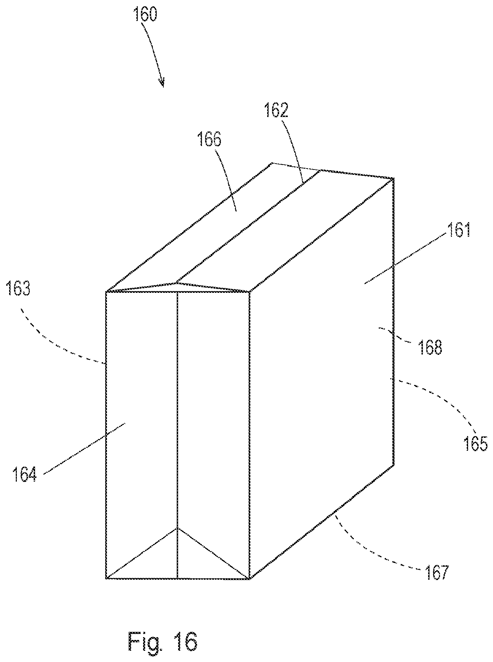

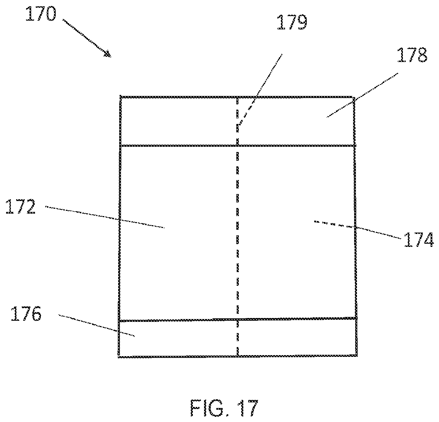

Referring to Fig. 16, packages of the present disclosure may be formed from a single sheet of packaging material suitably folded to form a bag structure 160. The packaging material 134 may form a front panel 161, a back panel 163 opposite the front panel 161, a first side panel 164, a second side panel 165 opposite the first side panel 164, a top panel 166, and a bottom panel 167 opposite the top panel 166, wherein the panels define an interior compartment 168 of the package, and wherein the one or more absorbent articles 132 are disposed in the interior compartment 168. The bag structure 160 and dimensions of the absorbent article 132 or stack of absorbent articles disposed therein may be suitably selected and effected through design, folding, stacking, compression and packaging processes such that the packaging material is taut about the absorbent article(s). Where the packaging materials comprise polymeric films, bonds forming any or all of the seams, such as seam 162, may be created by welding. (Herein, "weld" refers to a union between separate portions of film stock, effected by application of direct or indirect (e.g., ultrasonic) heating energy and pressure that causes separate portions of the film to at least partially melt and fuse together to some extent, forming a bonded area, joint or seam which cannot be separated without substantial destruction to the remainder of one or both joined portions.) Where the packaging materials comprise natural fibers, bonds forming any or all of the seams, such as seam 162, may be created by the application of adhesive. Other package shapes are contemplated, including flow wrap or horizontal form fill-and-seal wrap. Referring to Fig. 17, a flow wrap package 170 may comprise a first surface 172 and an opposing second surface 174. Rounded edges may be provided as a transition between the first surface 172 and the second surface 174. In another form, one or more fold lines may be provided between the first surface 172 and the second surface 174. The flow wrap package 170 may further comprise end seals 176 and 178, and a hoop seam 179 which may be disposed on the second surface 174. Flow wrap packages may be useful, particularly where a low number of absorbent articles are included within a package.

### Inks/Varnish:

The package of one or more absorbent articles of the present disclosure may comprise a component bearing a graphic. The component may be the package, a portion of the packaging material, and/or a component of the absorbent article, for example the backsheet, the secondary topsheet, the topsheet, and/or the landing zone. "Graphic" may include the depiction of a design or designs, any recognizable indicia such as a number, a letter, a word, a brand name, an icon, a logo, a character, a front/back indicator, a depiction of the absorbent article enclosed within the package, directions for use of the absorbent article and/or other consumer information, as well as a full flood of color across a surface, or a portion of a surface, of the component. "Graphics" may also include visible placement indicia to indicate where a sensor should be attached to the absorbent article, such as a dashed outline that matches the shape of a sensor, for example.

The graphic may be formed by the deposition of ink on at least a portion of the component. The ink may be any suitable ink known in the art. The ink may be a nitrocellulose-bases ink. Typical nitrocellulose-based ink formulations are described in U.S. Patent Application Publication No. 2008/0255275 A1 to Williams et al*.,* and U.S. Patent No. 6,548,572 to Breck et al. The ink may be a water-based ink. Water-based inks may be beneficial when the ink is disposed on the absorbent article, since the absorbent article may be in close contact with a wearer. Examples of water-based inks are set forth in U.S. Patent No. 10,836,196 to Warner et al.*,* and International Patent Application No. WO 2010/114899 A1 to Transvalidou et al. The ink may comprise pigment colorant particles. Pigment colorant particles may include but are not limited to, azo pigments, monoazo pigments, disazo pigments, azo pigment lakes, β-naphthol pigments, naphthol AS pigments, benzimidazolone pigments, disazo condensation pigments, metal complex pigments, isoindolinone and isoindoline pigments, quinacridone pigments, polycyclic pigments, phthalocyanine pigments, perylene and perinone pigments, thioindigo pigments, anthrapyrimidone pigments, flavanthrone pigments, anthanthrone pigments, dioxazine pigments, triarylcarbonium pigments, quinophthalone pigments, diketopyrrolo pyrrole pigments, titanium dioxide, iron oxide, and carbon blacks.

The ink may be applied to the component by any method known in the art. Specifically, the ink may be applied to the substrate using ink jet printers, flexographic printing presses, gravure printing presses, extrusion lamination, adhesive lamination, or a combination thereof.

The component of the present disclosure bearing a graphic may also comprise an overprint varnish. The overprint varnish may be applied to the component after application of the graphic. Use of an overprint varnish may be beneficial to protect the graphic from damage due to rubbing against packaging or other absorbent articles during manufacture, packaging, and shipping, and against wear during use. The overprint varnish, however, may also be vulnerable to interactions with volatile perfume composition components that diffuse across packaging materials. The volatile perfume composition components may interact with the overprint varnish, causing it to soften and lose its ability to protect the graphic from damage. The overprint varnish may comprise one of a very low Tg°C polymer, a polyurethane dispersion, a colloidal dispersion, a surfactant, talc, ammonia, water, and/or combinations thereof. Any other component described in the ink composition may also be useful in the OPV. The packaging material may include a polymeric film, an ink, and an overprint varnish.

The overprint varnish may be applied to the printed substrate by any method known in the art. The overprint varnish may be applied to the entire substrate, to only the ink-printed area, or to any combination thereof. The overprint varnish may be applied to the substrate using ink jet printers, flexographic printing presses, gravure printing presses, or a combination thereof. In one form, the overprint varnish is printed using an 80-100% screen printing plate. The overprint varnish may be printed after the inks are printed and allowed to dry.

### Examples

The following examples and comparative examples are provided to help illustrate the packaged absorbent articles described herein. The exemplified perfume compositions may be prepared by conventional formulation and mixing techniques. It will be appreciated that other modifications of the perfume compositions and packaging materials described herein within the skill of those in the formulation art may be undertaken. All parts, percentages, and ratios herein are by weight unless otherwise specified.

Diffusion rates of carrier components of perfume compositions are determined according to the Diffusion Rate Test Method disclosed herein. The Diffusion Rate Test Method calculates the rate of diffusion across various packaging materials.

Comparative Example 1 is a perfume composition comprising dipropylene glycol as a carrier component. Example 1 is a perfume composition comprising isopropyl myristate as a carrier component. The diffusion rates of the carrier components of Comparative Example 1 and Example 1 are tested according to the Diffusion Rate Test Method to determine the diffusion rate of the two carrier components across a polyethylene film devoid of ink and overprint varnish.

**Table 1. Diffusion of carrier components of perfume compositions across polyethylene film packaging material.**

| | Comparative Example 1 | Example 1 |
|---|---|---|
| | Dipropylene glycol | Isopropyl Myristate |
| Molecular Weight | 134 g/mol | 270.451 g/mol |
| Vapor Pressure (mmHg) | 0.0319 | 9.35x10⁻⁵ |
| Partition Coefficient (LogK_{ow}) | -1.07 | 7.71 |
| Water Solubility (mg/L at 20°C) | 6.96x10⁵ | 0.05 |
| Diffusion Rate over 2 hours at 40°C (ΔIₓ/t) | 0.1 | 0 |

As shown in Table 1, isopropyl myristate is water-insoluble, has a low vapor pressure, and exhibits little or no diffusion across the polyethylene film. Dipropylene glycol, on the other hand, is water-soluble, has a higher vapor pressure, and diffuses across the polyethylene film at a measurable rate. Without wishing to be bound by theory, it is believed that diffusion across a packaging material, such as a polyethylene film, may allow components of perfume compositions to interact with ink and/or overprint varnish disposed on the consumer-facing side of the packaging material. Therefore, selection of perfume composition components that do not diffuse, or diffuse at a very low level, across packaging materials may reduce or inhibit interaction of perfume composition components and the inks and/or overprint varnish disposed on packaging materials.

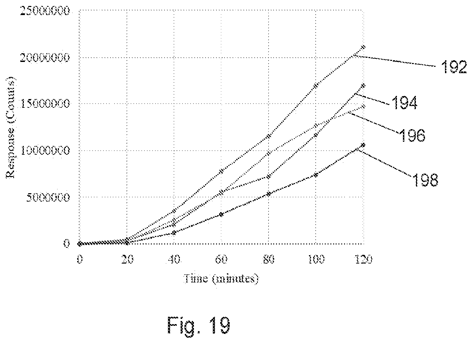

The diffusion rate of the perfume composition of Comparative Example 1 across polyethylene film devoid of ink and overprint varnish, with ink only, with overprint varnish only, and with both ink and overprint varnish is shown in Fig. 19. As shown in Fig. 19, total volatile compound diffusion across the polyethylene film decreased with the addition of ink and overprint varnish. Without wishing to be bound by theory, it is believed that the interaction of volatile compounds with both ink and overprint varnish is preventing a portion of the volatile compounds from reaching the headspace of the outer glass vial. It is further believed that this interaction of volatile compounds of the perfume composition with ink and/or overprint varnish may cause softening of the ink and/or overprint varnish, which may lead to scuffing or other damage to graphic disposed on the package of packaged absorbent articles.

Referring to Fig. 19, the diffusion rate of perfume composition of Comparative Example 1 was tested across polyethylene film devoid of ink and overprint varnish 192, polyethylene film with ink only 196, polyethylene film with overprint varnish only 194, and polyethylene film with both ink and overprint varnish 198. As shown in Fig. 19, the diffusion rate of perfume composition components across polyethylene film devoid of ink and overprint varnish 192 was greater than the diffusion rate of the other variables. The addition of ink or overprint varnish results in a decrease in the diffusion rate, indicating that the volatile compounds of the perfume composition interact with the ink and overprint varnish. Without wishing to be bound by theory, it is believed that volatile compounds from perfume compositions comprising a carrier component that is water soluble, and/or has a vapor pressure of less than about 7.5x10⁴ mmHg, and/or has a partition coefficient (log K_{ow}) of greater than 1.0 may not diffuse across some packaging materials, and/or may exhibit reduced or no interaction with the ink and overprint varnish disposed on packaging materials.

### Test Methods

### Diffusion Rate Test Method:

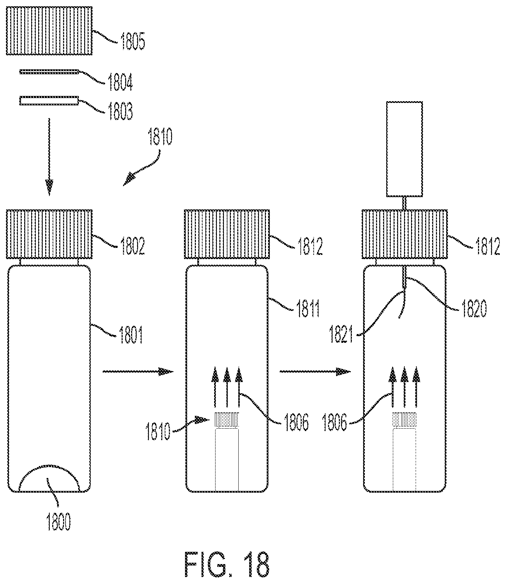

The Diffusion Rate Test Method calculates the rate of diffusion across various packaging materials. A schematic of the test set-up can be seen in Fig. 18. First, a perfume composition or components of a perfume composition 1800 are placed in a small gas chromatography vial 1801. The small vial is capped with a cap 1802 comprising a septum with a hole in the center 1803, a portion of a packaging material 1804, and a metal crimp cap 1805. The cap structure 1802 allows volatile compounds 1806 from the perfume composition 1800 to diffuse across the packaging material 1804. The small vial 1810 is placed inside a 40 mL glass vial 1811, and the glass vial 1811 is closed with a septum cap 1812.

Solid Phase Micro Extraction (SPME) is performed by inserting a needle 1820 containing an extracting-phase fiber 1821 through the septum of the 40 mL glass vial cap 1812. Volatile compounds 1806 are absorbed onto the extracting-phase fiber 1821 for a fixed time. The fiber is then inserted into a gas chromatogram injection port for analysis of volatile compounds in the headspace.

The dimensions and values disclosed herein are not to be understood as being strictly limited to the exact numerical values recited. Instead, unless otherwise specified, each such dimension is intended to mean both the recited value and a functionally equivalent range surrounding that value. For example, a dimension disclosed as "40 mm" is intended to mean "about 40 mm."

## Claims

1. A package (130) of one or more absorbent articles (132), comprising:
a packaging material (134) comprising a polymeric film and an ink;
an absorbent article (132) comprising a perfume composition, wherein the perfume composition comprises a perfume component and a carrier component, wherein the carrier component is from about 15 wt.% to about 85 wt.% of the perfume composition, and wherein the carrier component is insoluble in water,
wherein the carrier component comprises isopropyl myristate.

2. The package (130) of one or more absorbent articles (132) of claim 1, wherein the carrier component consists of isopropyl myristate.

3. The package (130) of one or more absorbent articles (132) of any one of the previous claims, wherein the polymeric film of the packaging material (134) comprises polyethylene.

4. The package (130) of one or more absorbent articles (132) of any one of the previous claims, wherein the packaging material (134) forms a front panel (161), a back panel (163) opposite the front panel, a first side panel (164), a second side panel (165) opposite the first side panel, a top panel (166), and a bottom panel (167) opposite the top panel (166), wherein the panels define an interior compartment (168) of the package (130), and wherein the one or more absorbent articles (132) are disposed in the interior compartment (168).

5. The package of one or more absorbent articles of any one of the preceding claims, wherein the packaging material consists of a polymeric film, an ink, and an overprint varnish.

6. The package of one or more absorbent articles of any one of the preceding claims, wherein the ink is a nitrocellulose-based ink.

7. The package of one or more absorbent articles of any one of claims 1-5, wherein the ink is a water-based ink.

8. The package of one or more absorbent articles of any one of the preceding claims, wherein the absorbent article comprises a topsheet, a backsheet, and an absorbent core disposed at least partially between the topsheet and the backsheet.

9. The package of one or more absorbent articles of claim 8, wherein a graphic is disposed on a garment-facing surface of the backsheet, and wherein the graphic is formed from a water-based ink.

10. The package of one or more absorbent articles of claims 8 or 9, wherein the absorbent article comprises a secondary topsheet disposed at least partially between the topsheet and the absorbent core, wherein a graphic is disposed on a wearer-facing surface of the secondary topsheet, and wherein the graphic is formed from a water-based ink.

11. The package of one or more absorbent articles of any one of the preceding claims, wherein the perfume component comprises beta-ionone.

12. The package of one or more absorbent articles of any one of the preceding claims, wherein the perfume component comprises 3-methyl-5-phenyl-1-pentanol (phenyl hexanol).

## Patentansprüche

1. Verpackung (130) eines oder mehrerer Absorptionsartikel (132), umfassend:
ein Verpackungsmaterial (134), umfassend eine Polymerfolie und eine Tinte;
einen absorbierenden Artikel (132), umfassend eine Duftstoffzusammensetzung umfasst, wobei die Duftstoffzusammensetzung einen Duftstoffbestandteil und einen Trägerbestandteil umfasst, wobei die Trägerbestandteil von etwa 15 Gew.-% bis etwa 85 Gew.-% der Duftstoffzusammensetzung ausmacht und wobei der Trägerbestandteil in Wasser unlöslich ist,
wobei der Trägerbestandteil Isopropylmyristat umfasst.

2. Verpackung (130) eines oder mehrerer Absorptionsartikel (132) nach Anspruch 1, wobei der Trägerbestandteil aus Isopropylmyristat besteht.

3. Verpackung (130) eines oder mehrerer Absorptionsartikel (132) nach einem der vorstehenden Ansprüche, wobei die Polymerfolie des Verpackungsmaterials (134) Polyethylen umfasst.

4. Verpackung (130) eines oder mehrerer Absorptionsartikel (132) nach einem der vorstehenden Ansprüche, wobei das Verpackungsmaterial (134) eine Vorderseite (161), eine Rückseite (163), die der Vorderseite entgegengesetzt ist, ein erstes Seitenfeld (164), ein zweites Seitenfeld (165), das dem ersten Seitenfeld entgegengesetzt ist, eine Oberseite (166) und eine Unterseite (167), die der Oberseite (166) entgegengesetzt ist, ausbildet, wobei die Felder ein Innenraumkammer (168) der Verpackung (130) definieren und wobei der eine oder die mehreren Absorptionsartikel (132) in der Innenraumkammer (168) angeordnet sind.

5. Verpackung eines oder mehrerer Absorptionsartikel nach einem der vorstehenden Ansprüche, wobei das Verpackungsmaterial aus einer Polymerfolie, einer Tinte und einem Überdrucklack besteht.

6. Verpackung eines oder mehrerer Absorptionsartikel nach einem der vorstehenden Ansprüche, wobei die Tinte cellulosenitratbasiert ist.

7. Verpackung eines oder mehrerer Absorptionsartikel nach einem der Ansprüche 1 bis 5, wobei die Tinte wasserbasiert ist.

8. Verpackung eines oder mehrerer Absorptionsartikel nach einem der vorstehenden Ansprüche, wobei der Absorptionsartikel eine Oberschicht, eine Unterschicht und einen Absorptionskern umfasst, der mindestens teilweise zwischen der Oberschicht und der Unterschicht angeordnet ist.

9. Verpackung eines oder mehrerer Absorptionsartikel nach Anspruch 8, wobei eine Grafik auf einer bekleidungsseitige Oberfläche der Unterschicht angeordnet ist und wobei die Grafik aus einer wasserbasierten Tinte ausgebildet ist.

10. Verpackung eines oder mehrerer Absorptionsartikel nach Anspruch 8 oder 9, wobei der Absorptionsartikel eine sekundäre Oberschicht umfasst, die mindestens teilweise zwischen der Oberschicht und dem Absorptionskern angeordnet ist, wobei eine Grafik auf einer trägerseitigen Oberfläche der sekundären Oberschicht angeordnet ist und wobei die Grafik aus einer wasserbasierten Tinte ausgebildet ist.

11. Verpackung eines oder mehrerer Absorptionsartikel nach einem der vorstehenden Ansprüche, wobei der Duftstoffbestandteil Beta-Ionon umfasst.

12. Verpackung eines oder mehrerer Absorptionsartikel nach einem der vorstehenden Ansprüche, wobei der Duftstoffbestandteil 3-Methyl-5-phenyl-1-pentanol (Phenylhexanol) umfasst.

## Revendications

1. Emballage (130) d'un ou plusieurs articles absorbants (132), comprenant :
un matériau d'emballage (134) comprenant un film polymère et une encre ;
un article absorbant (132) comprenant une composition de parfum, dans lequel la composition de parfum comprend un composant de parfum et un composant porteur, dans lequel le composant porteur représente d'environ 15 % en poids à environ 85 % en poids de la composition de parfum, et dans lequel le composant porteur est insoluble dans l'eau,
dans lequel le composant porteur comprend le myristate d'isopropyle.

2. Emballage (130) d'un ou plusieurs articles absorbants (132) selon la revendication 1, dans lequel le composant porteur est le myristate d'isopropyle.

3. Emballage (130) d'un ou plusieurs articles absorbants (132) selon l'une quelconque des revendications précédentes, dans lequel le film polymère du matériau d'emballage (134) comprend du polyéthylène.

4. Emballage (130) d'un ou plusieurs articles absorbants (132) selon l'une quelconque des revendications précédentes, dans lequel le matériau d'emballage (134) forme un panneau avant (161), un panneau arrière (163) opposé au panneau avant, un premier panneau latéral (164), un second panneau latéral (165) opposé au premier panneau latéral, un panneau supérieur (166) et un panneau inférieur (167) opposé au panneau supérieur (166), dans lequel les panneaux définissent un compartiment intérieur (168) de l'emballage (130), et dans lequel le ou les articles absorbants (132) sont disposés dans le compartiment intérieur (168).

5. Emballage d'un ou plusieurs articles absorbants selon l'une quelconque des revendications précédentes, dans lequel le matériau d'emballage est constitué d'un film polymère, d'une encre et d'un vernis de surimpression.

6. Emballage d'un ou plusieurs articles absorbants selon l'une quelconque des revendications précédentes, dans lequel l'encre est une encre à base de nitrocellulose.

7. Emballage d'un ou de plusieurs articles absorbants selon l'une quelconque des revendications 1 à 5, dans lequel l'encre est une encre à base d'eau.

8. Emballage d'un ou plusieurs articles absorbants selon l'une quelconque des revendications précédentes, dans lequel l'article absorbant comprend un feuillet supérieur, un feuillet inférieur et un noyau absorbant disposé au moins partiellement entre le feuillet supérieur et le feuillet inférieur.

9. Emballage d'un ou plusieurs articles absorbants selon la revendication 8, dans lequel un graphique est disposé sur une surface de la feuille arrière orientée vers le vêtement, et dans lequel le graphique est formé à partir d'une encre à base d'eau.

10. Emballage d'un ou plusieurs articles absorbants selon les revendications 8 ou 9, dans lequel l'article absorbant comprend un feuillet supérieur secondaire disposé au moins partiellement entre le feuillet supérieur et le noyau absorbant, dans lequel un graphique est disposé sur une surface orientée vers le porteur du feuillet supérieur secondaire, et dans lequel le graphique est formé à partir d'une encre à base d'eau.

11. Emballage d'un ou de plusieurs articles absorbants selon l'une quelconque des revendications précédentes, dans lequel le composant de parfum comprend de la bêta-ionone.

12. Emballage d'un ou de plusieurs articles absorbants selon l'une quelconque des revendications précédentes, dans lequel le composant de parfum comprend du 3-méthyl-5-phényl-1-pentanol (phényl hexanol).
